# EUROPEAN PATENT APPLICATION

(11) **EP 4 778 404 A1**
(43) Date of publication of application: **22.07.2026**
(21) Application number: 23952224.6
(22) Date of filing: 13.09.2023
(51) Int. Cl.: A23L 33/00, A61K 8/98, A61K 35/15, A61P 17/16, A61P 43/00, A61Q 19/08

(54) **ANTI-AGING PRODUCT, ANTI-AGING PREPARATION, AND PRODUCTION METHOD FOR SAME**

(71) Applicant: SOMA Gen-Ichiro, Setagaya-ku Tokyo 158-0084 (JP); Biomedical Research Group Inc., 158-0084 Tokyo (JP)
(72) Inventor: INAGAWA Hiroyuki, Takamatsu-shi, Kagawa 761-8062 (JP); KOHCHI Chie, Takamatsu-shi, Kagawa 761-8075 (JP); TANAKA Naoko, Ayauta-gun, Kagawa 761-2103 (JP); SOMA Gen-Ichiro, Tokyo 158-0084 (JP)
(74) Representative: Engelhard, Markus
(86) International application number: PCT/JP2023/033382
(87) International publication number: WO 2025/057330

(57) **Abstract**

The present invention provides: a product that rejuvenates aged cells or induces resistance to SASP and thereby inhibits aging; and a food, a cosmetic, a pharmaceutical, or the like that includes the product. Provided are: an anti-aging product that has anti-aging activity and is produced from macrophages stimulated with lipopolysaccharide; and an anti-aging preparation such as a food, a cosmetic, a skincare preparation, a supplement, a quasi-pharmaceutical, or a pharmaceutical that includes the anti-aging product. Also provided is a production method for the anti-aging product.

## Description

### [Technical Field]

The present invention relates to an anti-aging product, an anti-aging preparation, and a production method for same.

### [Background Art]

In 2019, the World Health Organization defined aging (excluding functional decline and dementia due to aging) as a disease in the 11th edition of the International Classification of Diseases. If aging is considered a disease, it becomes a target for prevention and treatment. The prevention or treatment of aging (rejuvenation) is collectively referred to as anti-aging. Currently, active research is being conducted on the actual state of senescent cells at the root of aging and the causes and mechanisms of aging, and various anti-aging approaches are being explored.

The causes of cellular aging are diverse. For example, aging can be induced by factors such as chronic inflammation, various stressors including oxidative and lysosomal stress, telomere abnormalities, and damage to genomic DNA. Therefore, sirtuins, which are involved in the repair of genomic DNA, have attracted attention, and NMN, a precursor of NAD (a coenzyme of sirtuins that decreases with aging), has gained attention as an anti-aging substance (NPL 1). However, the mechanism by which NMN prevents aging is still unclear, and it remains uncertain whether NMN truly has anti-aging effects.

Moreover, this approach has its challenges. It is unclear whether the decrease in NAD is a cause or a result of aging, and the idea of using NMN for anti-aging was conceived as a way to compensate for deficiencies. Therefore, even if oral NMN administration has some anti-aging effects, they are likely to be limited. Additionally, orally ingesting sufficient NMN to synthesize NAD is currently prohibitively expensive.

On the other hand, if there is a method to suppress aging by using the inherent abilities of cells that living organisms naturally possess, it would be possible to develop foods and pharmaceuticals that contribute to anti-aging in a broader sense, rather than simply compensating for deficiencies.

In senescent cells, proteins that inhibit the cell cycle, such as p16 (cyclin-dependent kinase inhibitor 2A) and p21 (cyclin-dependent kinase inhibitor 1), are increased (NPL 2, 3). In other words, senescent cells have stopped their cell cycle.

It is known that senescent cells induce SASP (Senescence-Associated Secretory Phenotype) and cause surrounding young cells to become senescent (NPL 4). Senescent cells continuously secrete inflammatory cytokines, chemokines, extracellular matrix-degrading enzymes, cell growth factors, and fragmented DNA, among others, packaged in exosomes and other vesicles into the extracellular space (NPL 5). These foreign substances in the body induce chronic inflammation throughout the body, leading to further increases in senescent cells. Therefore, the concept of senolytics, which removes senescent cells, has emerged as an anti-aging strategy (NPL 6).

The immune system monitors and attempts to eliminate not only foreign substances and cancer cells but also senescent cells. However, some senescent cells evade the immune system's surveillance and elimination mechanisms by expressing the immune checkpoint protein "PD-L1," which suppresses immune cell activity (NPL 7). In response, studies have been conducted to enhance the efficiency of immune-mediated elimination of senescent cells using anti-PD-1 antibodies (NPL 7). However, the limitation of this method lies in the fact that it assumes the immune system functions normally. Since immune cells themselves also age, it is difficult to expect efficient elimination of senescent cells by aged immune cells.

As described above, various approaches have been taken to inhibit the progression of aging, mainly consisting of methods to slow cellular senescence (such as NMN intake) and methods to quickly remove senescent cells (such as using anti-PD-1 antibodies). These approaches should be used in combination, but if it were possible to control aging (preventing aging or rejuvenation) by using the inherent abilities of biological cells, it would be possible to create foods and pharmaceuticals that contribute to anti-aging and rejuvenation in a broader sense than the two methods mentioned above. However, research on safely controlling the cellular capabilities of living organisms to rejuvenate aged cells or inducing resistance to SASP to inhibit aging has barely progressed.

Incidentally, we have previously demonstrated that oral administration of LPS (lipopolysaccharide) can prevent and improve various intractable diseases (NPL 8). In this research, we found that oral administration of LPS causes the nature of microglia in the brain to switch to a neuroprotective phenotype (NPL 8). Since LPS is not normally absorbed into the body, the phenotypic switching in microglia is not a direct effect of LPS. Therefore, the result suggests that LPS acts on innate immune cells such as mucosal macrophages, and then transmits signal to cells throughout the body via cell migration or secreted humoral factors. We refer to this system as the macrophage network (NPL 9). To confirm whether this system is useful for preventing or reversing aging, we conducted repeated studies to determine whether adding culture medium of LPS-stimulated macrophage to young cells exposed to the culture supernatant of aged fibroblasts or aged cells could induce rejuvenation or prevent aging.

As a result, we discovered that expression of p16 and p21, characteristic markers of senescent cells, decreases and cells become rejuvenated. Furthermore, we found that young cells, which would normally be accelerated in aging by the SASP of senescent cells, achieves prevention of aging when LPS-stimulated macrophage culture medium was added simultaneously. This led to the completion of the present invention.

### [Citation List]

### [Non Patent Literature]

[NPL 1] K. F. Mills et al., "Long-Term Administration of Nicotinamide Mononucleotide Mitigates Age-Associated Physiological Decline in Mice", Cell Metabolism, 2016.12, 24(6), p.795-806
[NPL 2] J. Krishnamurthy et al., "Ink4a/Arf expression is a biomarker of aging", The Journal of Clinical Investigation, 2004.11, 114(9), p.1299-1307
[NPL 3] X. Chen et al., "Senescence-like changes induced by expression of p21Waf1/Cip1 in NIH3T3 cell line", Cell Research, 2002.09, 12(3-4), p.229-233
[NPL 4] G. Nelson et al., "A senescent cell bystander effect: senescence-induced senescence", Aging Cell, 2012.02, 11(2), p.345-349
[NPL 5] Chika Misawa et al., "Function of Extracellular Vesicles Secreted by Senescent Cells," Drug Delivery System, 2021.06, 36(2), p.130-137
[NPL 6] Y. Zhu et al., "The Achilles' heel of senescent cells: from transcriptome to senolytic drugs", Aging Cell, 2015.03, 14(4), p.644-658
[NPL 7] T.W. Wang et al., "Blocking PD-L1-PD-1 improves senescence surveillance and ageing phenotypes", Nature, 2022.11, 611, p.358-364
[NPL 8] H. Mizobuchi, "Oral route lipopolysaccharide as a potential dementia preventive agent inducing neuroprotective microglia", Frontiers in Immunology, 2023.03.09, 14 https://doi.org/10.3389/fimmu.2023.1110583
[NPL 9] C. Kohchi et al., "Applications of Lipopolysaccharide Derived from Pantoea agglomerans (IP-PA1) for Health Care Based on Macrophage Network Theory", Journal of Bioscience and Bioengineering, 2006.12, 102(6), p.485-496

### [Summary of Invention]

### [Technical Problem]

The present invention provides a product that rejuvenates aged cells or inhibits aging by inducing resistance to SASP, etc., and also provides a food, a cosmetic, a pharmaceutical, etc. that contains the product.

### [Solution to Problem]

The anti-aging product of the present invention is produced from macrophages stimulated with lipopolysaccharide and having anti-aging effects.

The lipopolysaccharide may be a bacterium of the Enterobacteriaceae family.

The anti-aging preparation of the present invention comprises the anti-aging product.

The anti-aging preparation is preferably a food, a cosmetic, a skincare preparation, a supplement, a quasi-drug, or a pharmaceutical.

The anti-aging preparation is preferably in the form of a solid, liquid, gel, or aerosol.

The method for producing an anti-aging product of the present invention comprises stimulating macrophages with lipopolysaccharide to produce the anti-aging product having an anti-aging effect.

### [Advantageous Effects of Invention]

Conventionally, methods for slowing the progression of cellular aging and quickly removing senescent cells have been reported as anti-aging measures. In contrast, the present invention uses a factor group produced from macrophages stimulated with LPS to rejuvenate aged cells and to inhibit the aging of young cells caused by senescent cells.

### [Brief Description of Drawings]

[FIG. 1] Expression of each gene in each NB1RGB cell. p16 (A), p21 (B), Ki-67 (C) .
[FIG. 2] Expression of each gene in each NB1RGB cell. p16 (A), p21 (B), Ki-67 (C) .

### [Description of Embodiments]

Hereinafter, examples of the present invention will be described, but the present invention is not limited to the following examples.

### [Example 1]

### [Experiment of adding culture medium of LPS-stimulated macrophages to aged fibroblasts]

### (1) Materials and Methods

### 1) Cell Culture

Human skin fibroblast cell lines NB1RGB PDL14.9 (hereinafter referred to as "young cells") and PDL53.9 (hereinafter referred to as "old cells") (RIKEN BRC Cell Bank) were maintained in Minimum Essential Medium α (MEMα) containing 10% fetal bovine serum (FBS). Three days before the start of test, the medium was changed to Eagle's Minimum Essential Medium (EMEM) containing 10% FBS, and the test was conducted.

### 2) Preparation of conditioned medium

Human macrophage cell line THP-1 (ATCC) was cultured in RPMI-1640 containing 10% FBS. THP-1 was seeded at 1 × 10⁶ cells/mL, and LPS derived from Pantoea agglomerans (LPSp) was added to a final concentration of 100 ng/mL, and the culture supernatant was collected after 24 hours. This culture supernatant with LPSp added is referred to as THP/LPS-CM. As a control, the culture supernatant of THP-1 without LPSp added was also collected. This culture supernatant is referred to as THP-CM.

### 3) RNA extraction and real-time quantitative PCR (RT-qPCR)

Young cells (n = 3) and old cells (n = 9) were seeded in 6-well plates at 3 × 10⁵/3 mL/well. After 24 hours of pre-culture, the medium was replaced as follows:
* Young cell group: Three wells of young cells were replaced with fresh EMEM.
* Old cell group: Three wells of old cells were replaced with fresh EMEM.
* Old + THP-CM group: Another three wells of old cells were replaced with a 1:1 mixture of EMEM and THP-CM.
* Old + THP/LPS-CM group: Another three wells of old cells were replaced with a 1:1 mixture of EMEM and THP/LPS-CM.

Then, each was cultured for 24 hours.

Total RNA from young and old cells was extracted using the FastGene^{™} RNA Basic Kit (Nippon Genetics) according to the manufacturer's protocol. RNA was quantified by absorbance at 260 nm, and cDNA was synthesized using the TOYOBO ReverTra Ace^{™} qPCR RT Master Mix and rDNA Remover (TOYOBO Co., Ltd.). Primers for p16, p21, Ki-67, and GAPDH used in the experiment were prepared by Fasmac Co., Ltd. Ki-67 is a marker for proliferating cells.

RT-qPCR was performed using THUNDERBIRD^{™} SYBR^{™} qPCR Mix (TOYOBO Co., Ltd.) . PCR conditions were set as follows: reaction at 95°C for 1 minute, followed by 45 cycles of 95°C for 15 seconds and 60°C for 1 minute, and finally 95°C for 1 minute, 55°C for 30 seconds, and 95°C for 30 seconds. Glyceraldehyde-3-phosphate dehydrogenase (GAPDH) was used as an internal standard, and results are expressed as fold changes relative to young cells.

### 4) Statistical analysis

Statistical analysis of RT-qPCR was performed using BellCurve for Excel (ver. 4.04, Social Survey Research Information) to conduct ANOVA followed by Tukey-Kramer multiple comparison test. p < 0.05 was considered statistically significant.

### (2) Results

p16 and p21 are cyclin-dependent kinase inhibitors, are involved in progression of cell cycle and are each said to be one of aging markers. Both inhibit cell cycle when increased. Ki-67, a marker of proliferating cells, rejuvenates cells when increased. First, we examined the expression of three genes (p21, p16, Ki-67) in the young cell group, old cell group, old+THP-CM group, and old+THP/LPS-CM group.

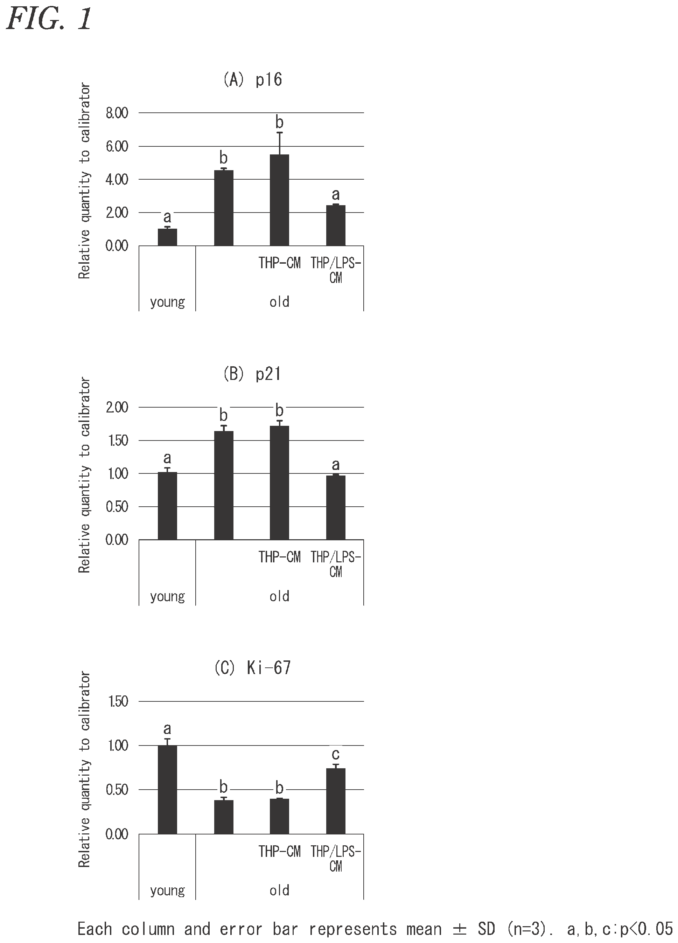

Results are shown in Fig. 1. p16 increased significantly in the old cell group and old+THP-CM group compared to the young cell group (p=0.001 and p<0.001, respectively). The old+THP/LPS-CM group reduced significantly compared to both the old cell group and old+THP-CM group (p=0.023 and p=0.003, respectively). No significant difference was observed between the young cell group and old+THP/LPS-CM group. No significant difference was observed between the old cell group and old+THP-CM group (Fig. 1A).

p21 increased significantly in the old cell group and old+THP-CM group compared to the young cell group (both p<0.001). The old+THP/LPS-CM group reduced significantly compared to both the old cell group and old+THP-CM group (both p<0.001). No significant difference was observed between the young cell group and old+THP/LPS-CM group. No significant difference was observed between the old cell group and old+THP-CM group (Fig. 1B).

Ki-67 was decreased significantly in the old cell group and old+THP-CM group compared to the young cell group (both p<0.001). The old+THP/LPS-CM group showed lower levels than the young cell group, but increased significantly compared to the old cell group and old+THP-CM group (both p<0.001). No significant difference was observed between the old cell group and old+THP-CM group (Fig. 1C).

From the above, it can be seen that aging is more advanced in the old cell group and old+THP-CM group than in the young cell group at the expression gene level. Addition of THP/LPS-CM reduced p16 and p21 and increased Ki-67 in old cells, bringing them closer to the state of young cells. There was no significant difference in the gene expression patterns of p16, p21, and Ki-67 24 hours after adding LPSp (final concentration 100 ng/mL) to the old cell group compared to the group to which nothing was added. These findings indicate that it is not LPS itself, but a factor secreted by LPS-stimulated macrophages that leads to cell rejuvenation.

### [Example 2]

[Experiment of adding culture medium of LPS-stimulated macrophages to young cells exposed to culture supernatant of aged fibroblasts after repeated division]

### (1) Methods

### 1) Cell culture and preparation of culture supernatant for aging induction

Human skin fibroblast cell lines NB1RGB PDL14.9 (hereinafter referred to as "young cells") and PDL61.9 (hereinafter referred to as "old cells") (RIKEN BRC Cell Bank) were maintained in Minimum Essential Medium α (MEMα) containing 10% FBS. For young cells, the medium was replaced with EMEM containing 10% FBS three days before the experiment. For old cells, the medium was replaced with EMEM containing 10% FBS, and their culture supernatant was collected after seven days. The culture supernatant was filtered through a 0.2 µm filter, and the filtrate (hereinafter referred to as "old sup") was used to induce aging in young cells.

### 2) Preparation of conditioned medium

Same as Example 1 (1) 2).

### 3) RNA extraction and real-time quantitative PCR (RT-qPCR)

Young cells (n = 21) were seeded in 6-well plates at 3 × 10⁵/3 mL/well. After 24 hours of pre-culture, the medium was replaced as follows:
* Old sup group: In 3 wells, the medium was replaced with old sup.
* Old sup + THP-CM group: In 3 wells, the medium was replaced with a 1:1 mixture of old sup and THP-CM (Ki-67 was not performed).
* 1, 10, 100, 1000 ng/mL groups: In 3 wells each, the medium was replaced with a 1:1 mixture of old sup and THP/LPS-CM at concentrations of 1, 10, 100, 1000 ng/mL (Ki-67 was performed only for 100 ng/mL).
* Young cell group: In the remaining three wells, the medium was replaced with EMEM as a control.

### All groups were cultured for 24 hours.

The primers used for total RNA and RT-qPCR were the same as those used in Example 1 (1) 3). However, RT-qPCR of Ki-67 was not performed.

### 5) Statistical analysis

Same as Example 1 (1) 4).

### (2) Results

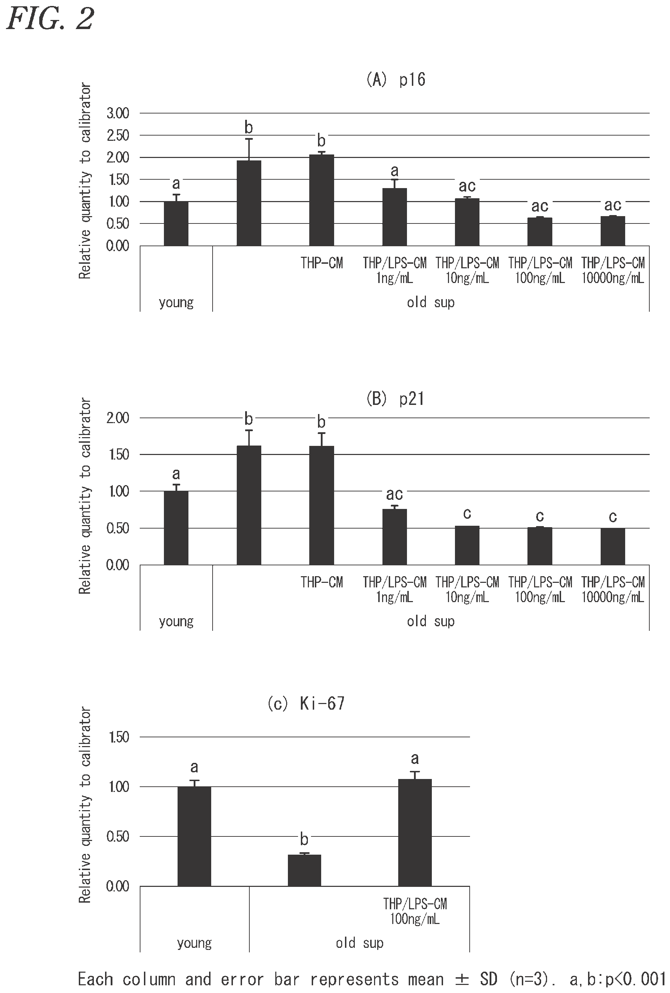

Figure 2 shows the expression of the genes for p16 and p21, which are said to be one of the aging markers, and Ki-67, a marker for proliferating cells.

p16 was significantly increased in the old sup group and the old sup+THP-CM group compared to the young cell group (p=0.003 and p<0.001, respectively) . All concentrations of old sup+THP/LPS-CM groups showed significant reductions compared to the old sup group and old sup+THP-CM group (all p<0.05). No significant difference was observed between the young cell group and old sup+THP/LPS-CM group. No significant difference was also observed between the old cell group and old sup+THP-CM group. Within the old sup+THP/LPS-CM groups, significant differences were observed between the 1 ng/mL group and the 100 ng/mL and 1000 ng/mL groups (all p<0.05), with reductions at the 100 ng/mL and 1000 ng/mL groups. No significant differences were observed when 10 ng/mL or more was added (Fig. 2A).

p21 was significantly increased in the old sup group and old sup+THP-CM group compared to the young cell group (both p<0.001). All concentrations of old sup+THP/LPS-CM groups showed significant reductions compared to the old sup group and old sup+THP-CM group (all p<0.001). Compared to the young cell group, significant differences were observed in the 10, 100, and 1000 ng/mL old sup+THP/LPS-CM groups (p=0.002, p=0.002, p=0.001, respectively), with reductions at the old sup+THP/LPS-CM groups. No significant differences were observed between the old cell group and old sup+THP-CM group. No significant differences were also observed between the old sup+THP/LPS-CM groups (Fig. 2B).

Ki-67 was significantly decreased in the old sup cell group compared to the young cell group (p<0.001). The old sup+THP/LPS-CM group showed a significant increase compared to the old sup cell group (p<0.001). No significant difference was observed between the young cell group and old sup+THP/LPS-CM group (Fig. 2C).

From the above, it can be seen that the gene expression levels of p16 and p21 increased in the old sup group and old sup+THP-CM group, indicative of advanced aging, compared to the young cell group. However, when THP/LPS-CM was added to young cells together with the culture supernatant of old cells, the expression of p16 and p21 was decreased and the expression of Ki-67 was increased. This is considered to be the result of THP/LPS-CM suppressing the aging of young cells caused by old cell culture supernatant. Additionally, the gene expression patterns of p16, p21, and Ki-67 24 hours after addition of old sup and LPSp (final concentration 100 ng/mL) to the young cell group showed no significant difference compared to the old sup cell group. These findings indicate that it is not LPS itself, but factors secreted by LPS-stimulated macrophages that suppress cellular aging.

### [Application Examples]

It can be seen from the above examples that anti-aging products with anti-aging effects can be produced by stimulating macrophages with lipopolysaccharides.

The action of lipopolysaccharides on macrophages is well-known for many lipopolysaccharides, and the lipopolysaccharides are not limited to that derived from Pantoea agglomerans, but may be derived from bacteria of the Enterobacteriaceae family.

The anti-aging preparation may be a food, cosmetic, a skincare preparation, a supplement, a quasi-pharmaceutical, or a pharmaceutical.

The anti-aging preparation may be in the form of a solid, liquid, gel, or aerosol.

All publications cited in this description are incorporated herein by reference in their entirety.

## Claims

1. An anti-aging product that is produced from macrophages stimulated with lipopolysaccharide and having an anti-aging activity.

2. The anti-aging product according to claim 1, wherein the lipopolysaccharide is a bacterium of the Enterobacteriaceae family.

3. An anti-aging preparation comprising the anti-aging product according to claim 1.

4. The anti-aging preparation according to claim 3, wherein the anti-aging preparation is a food, a cosmetic, a skincare preparation, a supplement, a quasi-pharmaceutical, or a pharmaceutical.

5. The anti-aging preparation according to claim 3, wherein the anti-aging preparation is in the form of a solid, liquid, gel, or aerosol.

6. A method for producing an anti-aging product, comprising stimulating macrophages with lipopolysaccharide to produce the anti-aging product having an anti-aging activity.
